# EUROPEAN PATENT APPLICATION

(11) **EP 4 424 687 A1**
(43) Date of publication of application: **04.09.2024**
(21) Application number: 23462001.1
(22) Date of filing: 03.03.2023
(51) Int. Cl.: C07D 489/12, A61P 25/04, A61K 31/485

(54) **NOVEL PROCESS FOR SYNTHESIZING OPIOID DERIVATIVES**

(71) Applicant: EUROAPI Hungary Limited Liability Company, 1045 Budapest (HU)
(72) Inventor: The designation of the inventor has not yet been filed
(74) Representative: Lavoix

(57) **Abstract**

The present application concerns a novel process suitable for synthesizing buprenorphine and the novel intermediates products involved by said process, as well as the buprenorphine and derivatives thereof obtainable by said process.

## Description

The present invention concerns a process for preparing buprenorphine and its derivatives.

Buprenorphine is an opioid used to treat opioid use disorder acute pain and chronic pain. Its hydrochloride salt is marketed under various trade names such as Subutex^{®}.

Buprenorphine is chemically known as 21-cyclopropyl-7a-(2-hydroxy-3,3-dimethyl-2-b 1)-6,14-endo-ethano-6,7,8,14-tetrahydrooripavine.

Various methods for the preparation of buprenorphine have been described (WO2010/039220, ). Precursor compounds to buprenorphine, such as thebaine, orvinol, display N- and O-methyl group that need to be removed (Machara et al. Advances in N- and O-demethylation of opiates, Targets in Heterocyclic Systems: Chemistry and Properties, Attanasi, O. A.; Merino, P.; Spinelli, D., Eds.;Società Chimica Italians: Rome, 2016; Vol. 20, 113-138). The demethylation steps, such as the N-demethylation, necessitate harsh conditions due to the inherent steric hindrance of the precursors. These harsh conditions consist in the use of strong reactants to reach the hindered methyl group, which present a low selectivity thus resulting in the production of residual impurities, and usually would require supplementary protective steps to protect other reactive groups, this with the overall impact on end product purity, an essential element of pharmaceutical active ingredient. Moreover, these reactants are usually toxic with the consequential health, environmental and safety drawbacks. As an illustration, WO 2021/151908 discloses the von Braun Reaction of orvinol using cyanogen bromide which impose special environmental, health and safety considerations while handling and the cleavage of cyanoamide intermediate requires harsh reaction conditions. Chloroformates as N-demethylation reagents are also hazardous and toxic chemicals (US 2009/0156815A1). US2011/0313163A1 describes Polonovsky-type reaction (Ac2O, Pd and Cu cat.) to form nor-orvinol from orvinol. Utilizing catalysts means that strong efforts should be made to identify and eliminate the risk of metal impurities of the final product. In case of using biocatalysts the removal of residual enzyme and cell debris must be done to avoid microbial contamination (Kutchan et. al. Nature Sustainability, 2019, 465-474). This in turn leads to a poor overall yield.

It is thus desirable to provide an improved method for preparing buprenorphine and derivatives thereof.

In particular, there remains a need to provide an efficient, industrially scalable process for the conversion of orvinol to buprenorphine and the derivatives thereof, with a high purity level, high yield, and improved safety, health and environmental impact.

Accordingly, the present invention is based on a breakthrough in the synthesis of buprenorphine and its derivatives, avoiding the above drawbacks and involving novel intermediate products.

In one of its objects, the present invention provides a process of preparation of buprenorphine or a derivative thereof of formula (I) : 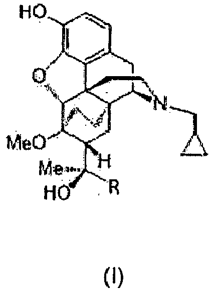 or a pharmaceutically acceptable salt thereof,
characterized in that said process involves the novel compound of formula (A) :

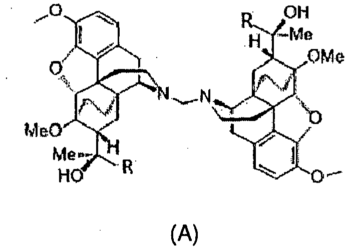

Where R is a C1-C6 alkyl.

When R is t-butyl, the compound of formula (A) may be called « orvinol dimer ».

Compound (A) so prepared can be recovered from the reaction mixture by conventional means. For example, compound (A) can be recovered by filtering or distilling the solvent from the reaction mixture, and/or by extraction. In addition, the product can, if desired, be further purified by various techniques, such as recrystallisation, reprecipitation or various chromatographic techniques, including column chromatography or preparative thin layer chromatography.

Derivatives of buprenorphine include in particular buprenorphine and diprenorphine

As used herein, « alkyl » refers to a linear or branched aliphatic hydrocarbon group where "branched" means that one or more lower alkyl groups such as methyl, ethyl or propyl are attached to one or more linear alkyl chain. Exemplary alkyl groups include methyl, ethyl, *n*-propyl, *i*-propyl, *n*-butyl, *t*-butyl, *n*-pentyl, 3-pentyl.

According to an embodiment, in Formula (I), R is preferably methyl or *t*-butyl (*tert-*butyl) of formula

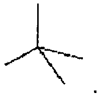

When R is preferably t-butyl, the compound of formula (I) is buprenorphine of formula (I-b):

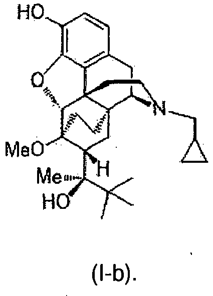

When R is methyl, the compound of formula (I) is diprenorphine of formula (I-d):

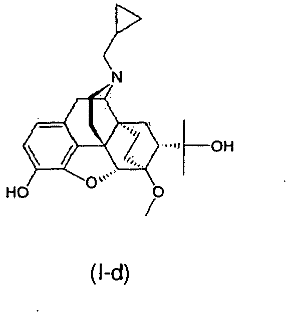

According to an embodiment, the process of preparation of a compound of formula (A) may comprise the steps of :
reacting orvinol or a derivative thereof of formula (VI) :

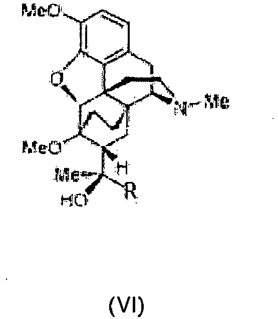

Where R is defined as above, it being understood that where R is *t*-butyl, said compound of formula (VI) is called herein orvinol, with an azodicarboxylate agent, such as diethyl azodicarboxylate, dibenzyl azodicarboxylate or diisopropyl azodicarboxylate, and reacting the obtained azodicarboxylate adduct in a protic, polar solvent, such as alcohols and/or water or optionally in the presence of aqueous bases, such as aqueous ammonia.

According to an embodiment, the azodicarboxylate agent is diisopropyl azodicarboxylate (DIAD, CAS RN 2446-83-5) of formula:

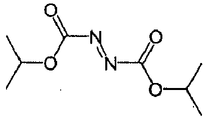

According to an embodiment, the azodicarboxylate adduct is of formula (V):

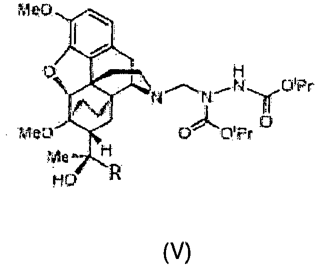

Where R is defined as above.

The reaction of the compound of formula (VI) with DIAD may be typically conducted in an organic solvent such as a polar aprotic solvent, such as acetonitrile (MeCN), acetone, THF.

It may typically be carried out under heating at a temperature comprised between the room temperature and the reflux temperature of the reactional mixture, typically around 60°C.

The reaction of the azodicarboxylate adduct of formula (V) to dimer may be typically conducted in a protic, polar solvent such as alcohols and/or water or optionally in the presence of aqueous ammonia and/or under heating at a temperature comprised between the room temperature and the reflux temperature of the reactional mixture, typically around 40°C.

As used herein, aqueous ammonia refers to NH₄OH.

According to an embodiment, the above two steps (the step from the compound of formula (VI) and the step from the adduct) may be conducted in sequence as separate reactions including separation of the adduct that is transiently formed, or alternatively as a one pot reaction (ie) without separation of said transient adduct that is formed.

Advantageously, the above two steps are conducted in one pot.

The compound (A) that is formed may be isolated from the reaction mixture, preferably by crystallization thereof, from alcohol, such as methanol.

According to a further embodiment, the process of preparation of the invention also comprises one or more prior step to prepare the compound of formula (A) as detailed above.

The compound of formula (VI) may be prepared by application or adaptation of known synthetic routes.

The compound of formula (I) may be prepared from the compound of formula (A). Accordingly, the process of the invention may further comprises a step of O-demethylation.

O-demethylation may be either conducted on the compound of formula (A), then followed by the cleavage of the dimeric structure, or after cleavage of the dimeric structure.

As a preferred embodiment, the compound of formula (II) may be prepared from the compound of formula (A). Accordingly, the process of the invention may further comprise the step of preparing the compound of formula (II) form compound (A). Said step may be typically carried out by O-demethylating and cleaving from the compound of formula (A) :

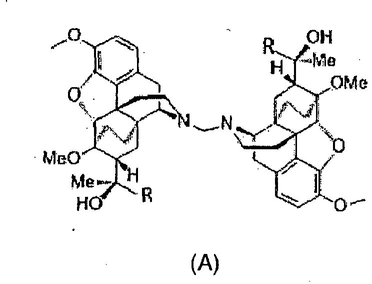

Where
R is defined as above.

O-demethylation of phenol-ethers are well known in the art and various conditions can be applied. (Machara et al. Advances in N- and O-demethylation of opiates, Targets in Heterocyclic Systems: Chemistry and Properties, Attanasi, O. A.; Merino, P.; Spinelli, D., Eds.;Società Chimica Italiana: Rome, 2016; Vol. 20, 113-138) According to an embodiment, the O-demethylation of compound (A) may be carried out by reacting the compound of formula (A) with a thiol compound in the presence of a base.

Preferably, said thiol compound is *t*-dodecanthiol (also called tert-dodecanthiol or tert-dodecyl mercaptan, CAS RN : 25103-58-6) of formula :

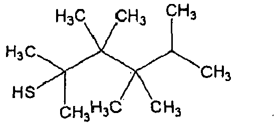

Preferably, the base may be chosen from organic and inorganic bases, preferably from strong bases, such as NaOt-Bu, KOt-Bu, NaH, C₂H₅ONa.

The O-demethylation reagents (the thiol compound and the base) may be preferably reacted in excess with respect to the compound of formula (A).

The O-demethylation step may advantageously be conducted in an organic solvent, preferably a polar aprotic solvent, such as dimethylformamide (DMF), N-methyl-2-pyrrolidone (NMP), N,N-dimethylacetamide (DMAc), dimethylsulfoxide (DMSO)

Typically, this reaction may be conducted under heating at temperatures generally comprised between 50°C and the reflux temperature of the reactional mixture, typically around 120-130°C.

According to an embodiment, cleavage of the dimer (A) or dimer (IV) to the corresponding monomers, such as compound of formula (II), may be conducted in the conditions of acidic hydrolysis, such as in the presence of an alcoholic solvent, such as MeOH, EtOH, iPrOH, preferably MeOH, in acidic conditions such as mixture of organic solvent and water, preferably in the mixture of alcohols and water with mineral acids (such as HCl, sulphoric acid, phosphoric acid or with organic acids (such as formic acid, acetic acid, citric acid, oxalic acid, tartaric acid, fumaric acid), preferably with HCl or tartaric acid.

Typically, suitable acidic conditions may be achieved at a pH comprised below 6. This may be advantageously achieved by using a suitable buffer, such as phosphate buffer, acetate buffer.

The O-demethylation step and the cleaving step may be conducted in sequence as separate reactions including separation of the intermediate product that is transiently formed, or alternatively as a one pot reaction. The term « one pot » as used herein refers to telescoped reactions, which are conducted in sequence without separation of transient intermediate products that may be formed in between the sequence.

According to an embodiment, the O-demethylation step and the cleaving step are conducted in a one pot reaction. Accordingly, the cleaving step may be conducted on the reaction mixture following the O-demethylation step without isolation of the intermediate product that is formed by the O-demethylation reaction. Said transient intermediate product obtainable following the O-demethylation step and prior to the cleaving step is of the following formula:

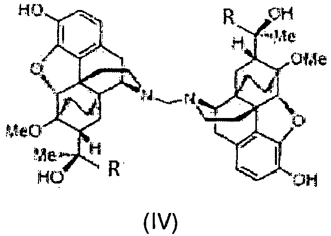

Where R is defined as above.

According to another embodiment, the cleaving step may be carried out on the isolated O-demethylated dimer of formula (IV) resulting from the O-demethylation step:

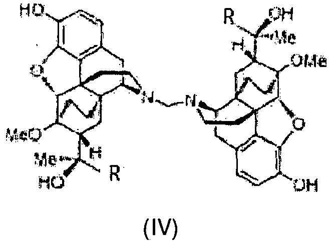

Where
R is defined as above.

Following the O-demethylation /cleaving steps the compound of formula (II) that is formed may be isolated from the reactional mixture, preferably by forming the hydrochloride salt thereof and crystallization thereof. The base form of the compound of formula (II) may be regenerated and released for conducting the next step of the process.

According to an embodiment, the process of the invention may further comprise the step of forming the compound of formula (I) by N-alkylating the compound of formula (II): 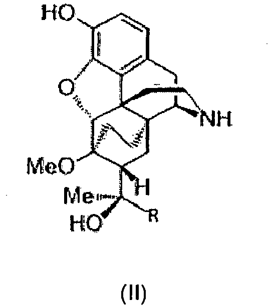 under appropriate conditions, such as reductive conditions, with a compound of formula (III) :

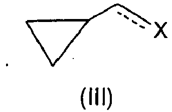

Where
R is as defined above;
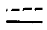 represents a single or double bond ; and
X is chosen from halogen atoms such as Chlorine (CI), Bromine (Br) or Iodine (I) atom, an oxygen atom, an hydroxy group, a tosylate group, a mesylate group, and a sulfonate group; provided that
when X is an oxygen atom, then 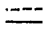 represents a double bond and
when X represents a halogen atom, an hydroxy group, a tosylate group, a mesylate group,
or a sulfonate group, then 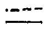 represents a single bond.
Halogen atom refers to a Chlorine (Cl), Bromine (Br) or Iodine (I) atom.

According to an embodiment, X is Br or O

According to an embodiment, the compound of formula (III) is chosen from the following compounds:

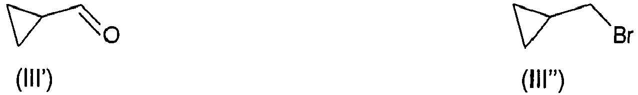

When compound (III) is of formula (III'), suitable reductive conditions include the use of a reductive agent such catalytic hydrogenation with Pd, Pt or Ni, borane complexes, including borane amine complexes such as borane-pyridine, borohydride exchange resin, AcOH in presence of Zn, preferably hydrides such as NaBH₃CN, NaBH₄ in presence of Mg(ClO₄)₂, Zn(BH₄)₂ in presence of ZnCl₂, NaBH₃CN with Ti(OiPr)₄ and more preferably NaBH(OAc)₃ (Sodium triacetoxyborohydride, also known as sodium triacetoxyhydroborate, commonly abbreviated STAB) Typically, the reductive agent may be used in excess with respect to the compound of formula (II).

The reductive alkylation may be advantageously conducted in any solvent compatible to the reductive agent, such as an organic solvent, such as a polar aprotic solvent, such as THF, dichloromethane,dichloroethane or polar protic solvent, such as alcohols at atemperature comprised between 0°C and the room tempertaure, or higher if needed.

Advantageous reactions include the use of a reductive agent such as NaBH(OAc)₃, in THF.

When compound (III) is of formula (III"), suitable conditions wellknown in the art for alkylating an amine may be used.

The reductive alkylation of a compound of formula (II) generally leads to a compound of formula (I) in the form of a base. If desired, the process of the invention may also include the additional step of converting the compound of formula (I) in the base form into its salified form.

According to an embodiment, the process of the invention may thus further include the additional step of forming a pharmaceutically acceptable salt from the compound of formula (I).

Acid addition salts may typically be formed with the compound of formula (I) in which the basic dialkylamino function is present.

The pharmaceutically acceptable salts generally refer to nontoxic, acid addition salts. The salts may generally be chosen to be compatible with the storage requirements, the customary pharmaceutical vehicles and/or adapted for the desired administration route. Acid addition salts of the compounds useful according to this invention may be prepared by reaction of the free base with the appropriate acid, by the application or adaptation of known methods. For example, the acid addition salts of the compounds useful according to this invention may be prepared either by dissolving the free base of compound of formula (I) in water or aqueous alcohol solution such as isopropanol or other suitable solvents containing the appropriate acid and isolating the salt so formed by cooling, evaporating the solution, or by reacting the free base of compound of formula (I) and the desired acid in an organic solvent, in which case the salt may separate directly or can be obtained by concentration of the solution.

Some suitable acids for use in the preparation of such salts are hydrochloric acid, hydrobromic acid, phosphoric acid, sulfuric acid, various organic carboxylic and sulfonic acids, such as acetic acid, citric acid, propionic acid, succinic acid, benzoic acid, tartaric acid, fumaric acid, mandelic acid, ascorbic acid, malic acid, methanesulfonic acid, toluenesulfonic acid, fatty acids, adipate, alginate, ascorbate, aspartate, benzenesulfonate, benzoate, cyclopentanepropionate, digluconate, dodecylsulfate, bisulfate, butyrate, lactate, laurate, lauryl sulfate, malate, hydroiodide, 2-hydroxy-ethanesulfonate, glycerophosphate, picrate, pivalate, pamoate, pectinate, persulfate, 3-phenylpropionate, thiocyanate, 2-naphthalenesulfonate, undecanoate, nicotinate, hemisulfate, heptonate, hexanoate, camphorate, camphersulfonate, and others. Preferably, the acid is hydrochloric acid.

Conversely, if needed the compounds of formula (I) can be also regenerated from their salts by the application or adaptation of known methods. For example, compounds of formula (I) can be regenerated from their acid addition salts by treatment with an alkali, e.g. aqueous sodium bicarbonate solution or aqueous ammonia solution.

According to a preferred embodiment, the invention provides a process for the preparation of a compound of formula (I) or any salts thereof characterized in that said process involves the following steps:
- N-demethylation of compound of formula (VI) to produce compound of formula (A),
- Then O-demethylation of compound of formula A followed by a cleavage step to produce compound of formula (II), and
- N-alkylation of compound of formula (II).

According to a particularly preferred embodiment, the invention provides a process for the preparation of a compound of formula (I) or any salts thereof characterized in that said process involves the following steps:
- N-demethylation of compound of formula (VI) with diisopropyl azodicarboxylate in acetonitrile to produce compound of formula (A),
- Then O-demethylation of compound of formula (A) with tert-dodecanthiol (also called tert-dodecyl mercaptan) following by a cleavage step in methanol water mixture with HCl to produce compound of formula (II), and
- N-alkylation of compound of formula (II) with compound of formula (III').

According to an even more preferred embodiment, in the above steps described above, R is t-butyl in formulae (A), (IV) and (I).

The intermediates of formula (IV) and (V): 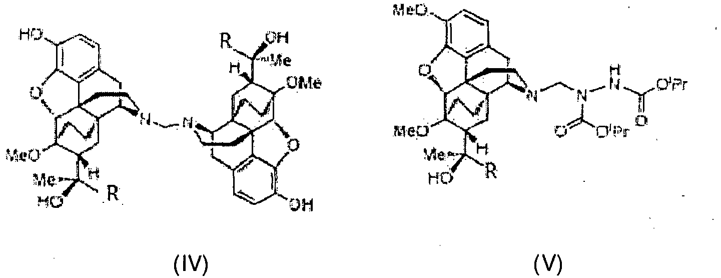 where R is defiend as above involved by the above processes are novel.

Said compounds of formula (IV) and (V) *per se* are thus still further object of the present invention

As used herein and unless indicated above:
the above reactions can generally take place over a wide range of temperatures, and the precise reaction temperature may not be critical to the invention;
typically, the time required for the above reactions may also vary widely, depending on many factors, notably the reaction temperature and the nature of the reagents. However, provided that the reaction is effected under the preferred conditions outlined above, a period of from about 3 hours to about 20 hours will usually suffice;
various solvents may be employed, provided that they have no adverse effect on the reactions or on the reagents involved. Typical examples of suitable solvents include: hydrocarbons, which may be aromatic, aliphatic or cycloaliphatic hydrocarbons, such as hexane, cyclohexane, benzene, toluene and xylene; amides, especially fatty acid amides, such as dimethylformamide; and ethers, such as diethyl ether and tetrahydrofuran.

In the above process, starting compounds and reactants, unless otherwise indicated, are commercially available or described in literature, or can be prepared according to methods described in literature or known to one of skill in the art.

The compound thus prepared may be recovered from the reaction mixture by conventional means. For example, the compounds may be recovered by distilling off the solvent from the reaction mixture or, if necessary after distilling off the solvent from the reaction mixture, pouring the residue into water followed by extraction with a water-immiscible organic solvent and distilling off the solvent from the extract. Additionally, the product can, if desired, be further purified by various well-known techniques, such as crystallization, recrystallization, reprecipitation or the various chromatography techniques, notably column chromatography or preparative thin layer chromatography.

According to a further object, the invention also concerns buprenorphine or a derivative thereof obtainable by the process of the invention, or an addition salt thereof, as detailed above. They are hereafter called « compounds according to the invention ».

According to a further embodiment, a compound according to the invention has a purity of at least 95%, preferably at least 98%, more preferably at least 98.5%, even more preferably at least 99%, said percentage being expressed in weight of said buprenorphine or derivative with respect to the total weight of the product obtained by said process.

According to the process of the invention, buprenorphine or said derivative is typically obtained with a yield greater than 55%, preferably greater than 60%, preferably greater than 64%, more preferably greater than 70% and even more preferably greater than 73%, said yield being expressed in the molar quantity of the product obtained, respective to the molar quantity of the starting product, preferably orvinol.

The compounds of formula (I) are typically ligands of the opioid receptor. They can be useful for the preparation of medicaments targeting said receptor.

The compound according to the invention is suitable for use as an active pharmaceutical ingredient ("API") which may be defined, in the broadest terms, as a chemical entity that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a pharmaceutical composition can include at least one API, or combinations thereof, in various types of formulations, for the treatment of one or more diseases.

Therefore, another object of the invention is a pharmaceutical composition comprising a compound according to the invention or a salt thereof, and a pharmaceutically acceptable excipient.

The terms "pharmaceutical formulation", "drug", "medicament" are used synonymously herein.

These pharmaceutical compositions generally comprise an effective dose of at least one compound according to the invention, and at least one pharmaceutically acceptable excipient.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of a compound according to the invention, which is required to achieve the desired biological effect will vary depending upon a number of factors, including the dosage of the drug to be administered, the chemical characteristics (e.g., hydrophobicity) of the compounds employed, the potency of the compounds, the type of disease, the diseased state of the patient, and the route of administration.

The identification of those subjects who are in need of treatment of herein-described diseases and conditions is well within the ability and knowledge of one skilled in the art. A clinician skilled in the art can readily identify, by the use of clinical tests, physical examination and medical/family history, those subjects who are in need of such treatment.

The pharmaceutical compositions may conveniently be administered in unit dosage form and may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000.

The compounds of the present invention are capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition, as described hereinafter.

Said excipients are chosen according to the pharmaceutical form and the administration route desired, among usual excipients known of one of skill in the art.

According to an embodiment, the pharmaceutical composition may be suitable for oral, sublingual, or transdermal administration or for injection.

The appropriate unitary dosage forms for oral or sublingual administration comprise tablets, hard or soft gelatin capsules, powders, granules, lozenges, pastilles and oral solutions or suspensions.

The appropriate unitary dosage forms for transdermal administration include the creams, gels, ointments, lotions, paste, gel, spray, aerosol, oil, Formulations suitable for transdermal administration can be presented as discrete patches.

The appropriate unitary dosage forms for injection include sterile aqueous or nonaqueous solutions, suspensions, and emulsions.

These pharmaceutical compositions are useful in therapeutics, in particular as an analgesic or for opioid use disorder.

A compound of formula (I) such as buprenorphine obtainable by the process of the invention, or a salt thereof for use for treating and/or preventing opioid use disorder, acute pain and/or chronic pain is thus another object of the invention.

The present invention, according to another of its objects, also relates to a method for the treatment of the above pathologies, which comprises the administration to a patient of an effective dose of a compound according to the invention

The preferred dosage of the compound according to the invention to be administered is likely to depend on such variables as the type and extent of progression of the disease or disorder, the overall health status of the particular patient, the relative biological efficacy of the compound selected, and formulation of the compound excipient, and its route of administration.

Typically, said buprenorphine may be administered at a dose comprised between 2 and 24 mg a day.

In specific cases, higher or lower dosages may be appropriate; notably depending on the mode of administration, such as intravenous dosage may be administered between 0.3 to 0.6mg every 6 hours, these dosages are comprised within the scope of the present invention. According to usual practice, the dosage suitable to each patient is determined by the physician according to the administration route, the weight and response of the patient.

The compounds of the current invention can be employed as the sole active ingredient in a pharmaceutical composition. Alternatively, they can be used in combination or combined with other pharmaceutical agents associated with the same or other disease states. Said combinations can be administered simultaneously or separately.

Thus, as a still further object, the present invention also concerns a combination comprising buprenorphine obtainable by the process of the invention, and an opioid antagonist, such as naloxone, for simultaneous, separate or staggered over time administration.

The compounds of the invention, their methods or preparation and their biological activity will appear more clearly from the examination of the following examples which are presented as an illustration only and are not to be considered as limiting the invention in its scope.

The following examples describe the synthesis of some compounds according to the invention. These examples are not intended to be limitative and only illustrate the present invention. The numbers of the exemplified compounds refer to those in the table given later, which illustrate the chemical structures and the physical properties of a number of compounds according to the invention.

### Figures

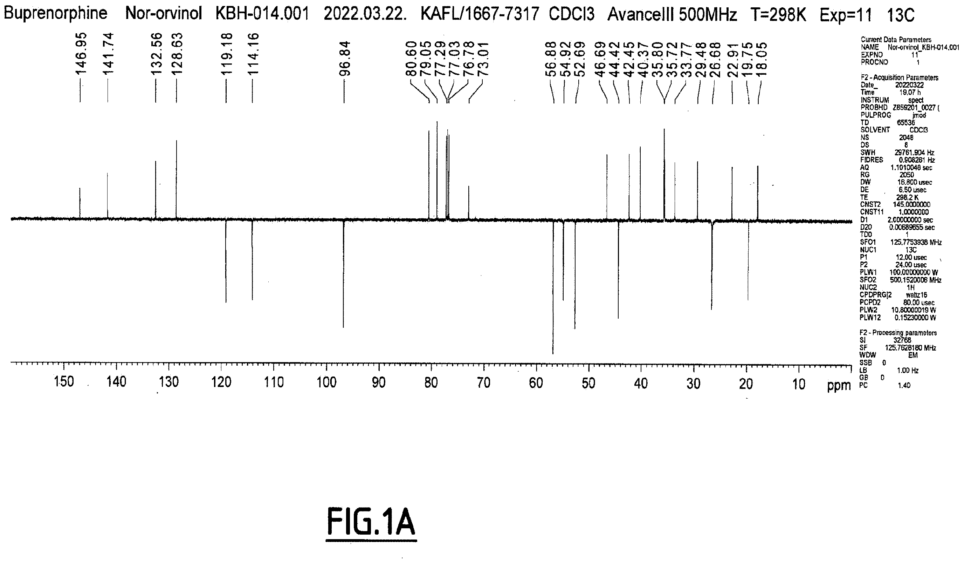

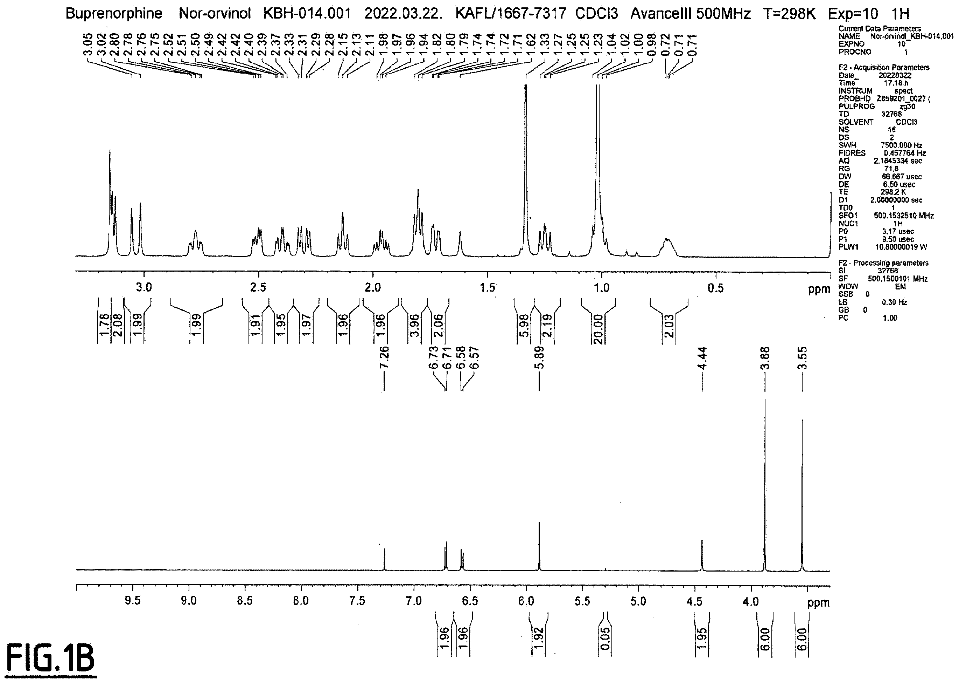

Figure 1 represents the NMR graph of compound (A): ¹H NMR (Figure 1A) and 13C NMR (Figure 1B).

### Examples

### Synthesis of orvinol dimer (A) from orvinol (VI):

The procedure was performed as follows:
8.84 g (20 mmol) of orvinol base is suspended in 44 mL (5V) of acetonitrile, 4.76 mL (24 mmol, 1.2 eq) of diisopropyl azodicarboxylate is added and the reaction mixture is stirred at 60 °C for 14-18 hours. The resulted solution is cooled to room temperature, 48 mL of methanol then 5.6 mL of 25 % aq. ammonia solution (4 eq) are added, and the mixture is stirred at 40 °C for 14-16 hours. The product starts to precipitate after a short time even at this temperature. The reaction is cooled to 0 °C, stirred at this temperature for 2 hours then the product is filtered and dried. The isolated product is dissolved in 80 mL of dichloromethane. The opalescence solution is filtered (~ 200 mg of yellow precipitate is filtered off) then the filtrate is evaporated to dryness. The residue (white foam) is orvinol-dimer that can be suspended in 40 mL of methanol and then filtered for better handling.

The following results were obtained (Table 1):

| | **Amount** | **Yield** | **Purity** * | **Analysis** |
|---|---|---|---|---|
| | 1.8 g | 82 % | 99.9 % | NMR, LC-MS, HPLC |
| | 7.2 g | 84 % | 99.9 % | NMR, HPLC, MS-TOF |
| ** | 7.0 g | 81 % | 99.2 % | NMR, LC-MS, HPLC |
| | 7.3 g | 84 % | 99.7 % | NMR, LC-MS, HPLC |
| *** | 7.9 g | 91 % | 99.9 % | LC-MS, HPLC |
| | 7.1 g | 82 % | 99.9 % | NMR, HPLC |

| | | | | |
|---|---|---|---|---|
| * HPLC / LC-MS: corresponds to nor-orvinol (M: 427g/mol) **: adduct formation with 1.1 eq of DIAD ***: final treatment with methanol was omitted NMR measurements of all selected lots gave the same spectrum. | | | | |

Results in Table 1 show that the current process for the preparation of orvinol-dimer is robust, the product is isolated in good yield with excellent purity.

Formation of orvinol-DIAD-adduct (V):

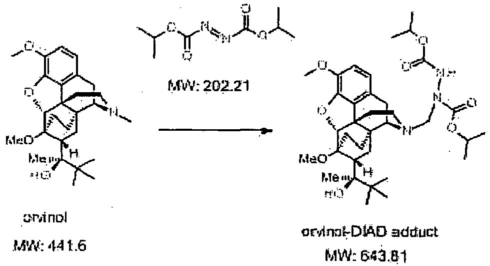

Orvinol was reacted with diisopropyl azodicarboxylate (DIAD) to form the adduct.

The adduct further reacted to form a d dimer-type new compound from orvinol-DIAD-adduct, , corresponding to an aminal derivative, for which the structure was:

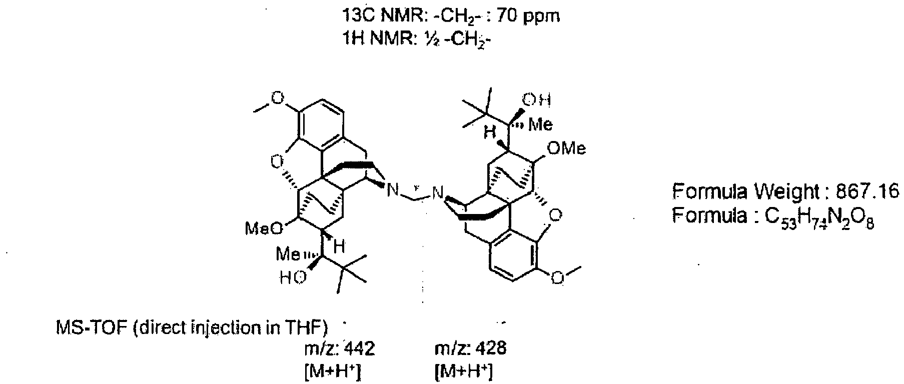

This was demonstrated by NMR and further indirect evidence for the proposed structure came from MS-TOF assay, which avoids acidic treatment of the sample and detects two fragments of the molecule but not the molecule ion. Thermal characteristics of orvinol-dimer and nor-orvinol were compared by DSC/TG showing significant difference between the two compounds.

Solubility properties of orvinol-dimer:
The product is freely soluble in DCM, moderately in THF and practically insoluble in alcohols, ACN, acetone.

HPLC / LC-MS: sample preparation and elution under acidic condition resulted in the dimer hydrolysed to nor-orvinol (M+H+: 428), so only this single peak is detectable.

### NMR of orvinol-dimer:

### Materials and method

NMR spectra were recorded on Bucker Avance III 500 MHz nmr spectrometer at 298 K in Chloroform-d.

### NMR Peaks:

¹H NMR (CDCl₃, 500 MHz) δ (ppm): 6.72 (d, 2H, *³J* = 8.0 Hz), 6.57 (d, 2H, *³J* = 8.0 Hz) 5.89 (s, 2H, OH), 4.44 (s, 2H, OCH), 3.88 (s, 6H, OCH₃), 3.55 (s, 6H, OCH₃), 3.15 (s, 2H, NCH₂N), 3.13 (d, 2H, *³J* = 6.5 Hz), 3.03 (d, 2H, ²*J* = 18.5 Hz), 2.78 (td, 2H, *³J* = 3.5 Hz), 2.51 (dd, 2H, *²J* = 11.5 Hz, *³J* = 5.0 Hz), 2.39 (td, 2H, *³J* = 12.2 Hz), 2.30 (dd, 2H, *²J* = 18.5 Hz, *³J* = 6.5 Hz), 2.13 (t, 2H, *³J* = 9.8 Hz), 1.96 (td, 2H, *³J* = 6.00 Hz), 1.80 (t, 4H), 1.73 (dd, 2H, *²J* = 12.5 Hz, *³J* = 2.0 Hz), 1.33 (s, 6H, CH₃), 1.25 (2H), 1.02 (s, 18H, ^{t}Bu-CH₃), 1.00 (2H), 0.71 (m, 2H)

¹³C NMR (CDCl₃, 126 MHz) δ (ppm): 146.9, 141.7, 132.6, 128.6, 119.2, 114.2, 96.8, 80.6, 79.1, , 73.0, 56.9, 54.9, 52.7, 46.7, 44.4, 42.5, 40.4, 35.8, 35.7, 33.8, 29.5, 26.7, 22.9, 19.8, 18.0.

The NMR spectra are illustrated in Figure 1 A-B.

### Synthesis of buprenorphine from orvinol-dimer

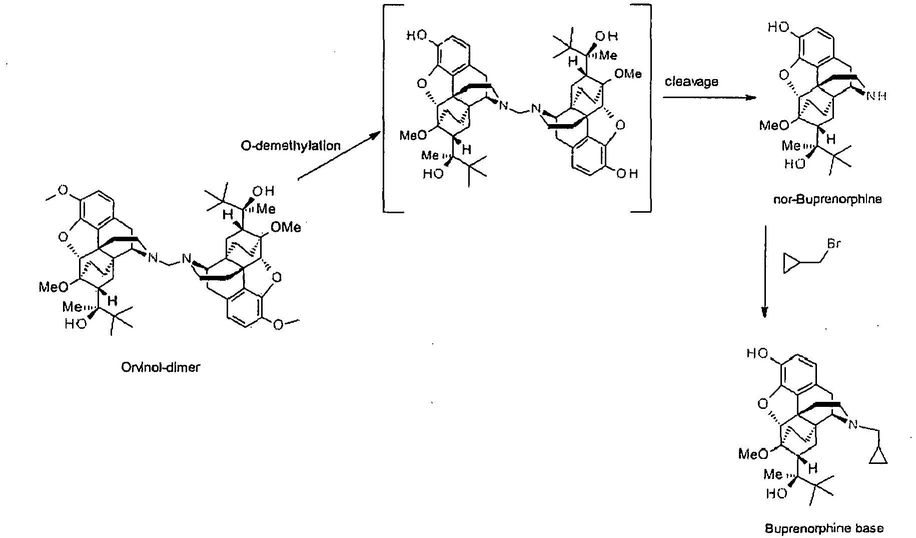

### O-demethylation of orvinol-dimer:

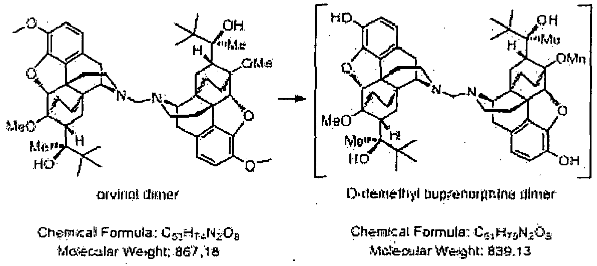

The reaction was performed according to the steps listed in Table 3 :
4.0 g of Orvinol-dimer (4.6 mmol) is suspended in 32 mL of DMF then 5.43 mL (23.06 mmol, 5 eq) of t-dodecanethiol followed by 2.22 g (23.06 mmol, 5 eq) sodium-t-butoxide are added. The mixture is stirred at 70-75 °C for 15 minutes under a slow nitrogen flow, then the temperature is raised to 125-129 °C. The initial suspension starts to dissolve to yield an opalescent solution then precipitation is observed. After 6 hours reaction time (conversion by HPLC: > 99 %) the suspension is cooled to 20 °C, quenched with a mixture of 100 mL water and 75 mL of ethylacetate. The aqueous phase is washed with 30 mL of ethylacetate. To the combined phase 50 mL of water is added and the pH is set to 1.5 by 85 % phosphoric acid. The organic phase is washed with 20 mL of water. The combined aqueous phases are washed with 30 mL of cyclohexane. The pH is adjusted to 9.5 by addition of 25 % aq. ammonia solution, then extracted with dichloromethane. The organic phase is washed with water, dried with Na₂SO₄ and evaporated to dryness.

The isolated product: 4.0 g off-white solidifying thick oil (quantitative yield) as O-demethyl-buprenorphine-dimer (crude).

### Cleavage of O-demethyl-buprenorphine-dimer to nor-buprenorphine

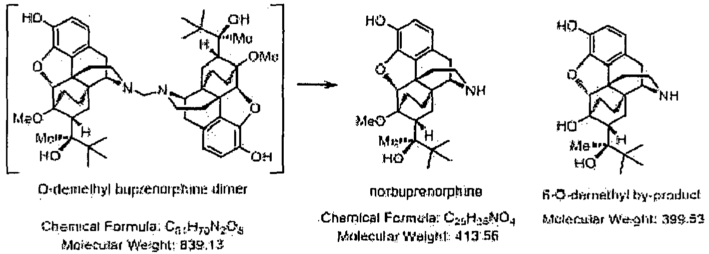

Hydrolysis was conducted on the isolated O-demethylated dimer.

Transformation to pure monomer formation was successfully performed by treating the dimer dissolved in methanol with aq. hydrochloric acid. Based on NMR investigation the precipitated product proved to be the desired monomer.

3.6 g of the crude product from the previous step is dissolved in 36 ml of methanol at ~40 °C then 30 mL of 1N HCl (~4 eq) is added, and the solution is stirred at 40 °C for 2 hours. The volume is reduced to ~1/3 part (45 °C / 60 mbar) then the mixture is cooled to room temperature and stirred overnight. The resulted suspension is stirred at 0 °C for an hour then filtered off and dried. The isolated product: 3.3 g white crystals as nor-Buprenorphine x HCl (yield: 88 %) NMR: corresponds to monomer. From 3.0 g of the isolated salt nor-Buprenorphine base is released with cc. ammonia solution for the subsequent step.

The isolated product: 2.74 g white crystals as nor-Buprenorphine (yield: quant.) Purity (HPLC): 98.8 %

### Buprenorphine synthesis from nor-Buprenorphine

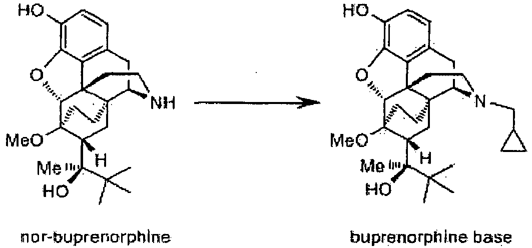

Last chemical step on this synthesis line is introduction of cyclopropylmethyl group preferably by direct alkylation or reductive alkylation, that have been previously described in the art (See Table 6)

| **Method** | **Reagent** | **Conditions** | **Yield** | **Ref.** |
|---|---|---|---|---|
| Alkylation | 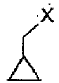 | X = Br | 56% | WO2007/81506 |
| | | Na₂CO₃, DMF | | |
| | | 80...100 °C, 4 h | | |
| | | X = Br | 94% | US2010181820 |
| | | NaHCO₃, K), DMF | | |
| | | 85 °C, 5.5 h | | WO2010/39220 |
| | | X = Br | 86% | *Adv. Synth. Catal.* **2012**, *354*, 613. |
| | | NaHCO₃, NMP | | |
| | | 80 °C, 18 h | | |
| | | X = Br | 86% | WO2021/151908 |
| | | DIPEA, DMF | | |
| | | 70 °C, 7 h | | |
| | | X = Cl | 85% conv. | US20111269964 |
| | | NaHCO₃, Na₂CO₃, NaBr | | |
| | | *N*-methylpyrrolidinone, H₂O | | |
| | | 70 °C, 5 h | | |
| Reductive alkylation | 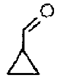 | [Ru(*p*-cymene)Cl₂]₂, | 89% | WO2009/12005 |
| | | (1S, 2S)-(+)-N-tosyl-diphenylethylenediamine | | |
| | | HCOOH, Et₃N, MeCN | | |
| | | 20°C, 36 h | | |
| | | [Ru(*p*-cymene)Cl₂]₂, HCOOH, Et₃N, MeCN | 78% | WO2018/211331 |
| | | 20...60 °C, 1 h | | WO2021/144362 |
| | | | | US2021/230655 |
| | | Hz, PUC, NMP, MeOH | 64% | WO2006/35195 |
| | | 50°C, 2 h | | |

A novel route of buprenorphine by reductive alkylation has been identified as follows:

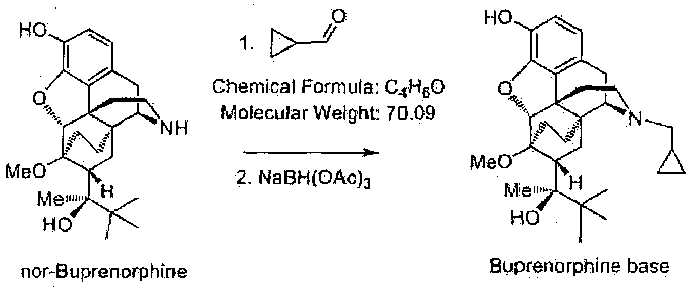

For reductive alkylation with sodium triacetoxyborohydride (NaBH(OAc)₃) reductive agent has no reference in the literature on nor-buprenorphine, therefore the detailed procedure is described below:
1.28 g (3.1 mmol) of nor-Buprenorphine base is dissolved in 38 mL of THF at 0 °C, then 0.46 mL (2 eq) of cyclopropylcarbaldehyde and 71 µL (0.4 eq) of AcOH, then 3.28 g (5 eq) of NaBH(OAc)₃ are added and the reaction mixture is stirred at 0-5 °C for 30 minutes. The temperature is raised to 25 °C and another portion of reductive agent (3.28 g) is added then the stirring is continued for an additional 3 hours. HPLC of the reaction mixture shows complete conversion. The reaction mixture is poured to the mixture of 100 mL of water and 50 mL of dichloromethane and the pH is set to 9.5 by addition of cc. ammonia solution. The aqueous phase is extracted with 2 × 50 mL of dichloromethane then the combined organic phase is washed with water, dried, and evaporated to dryness.
The isolated product: 1.32 g white crystals as Buprenorphine base (crude)
Yield: 91 %
Purity (HPLC): 99.1 %

The above process thus represents a new route to synthesize buprenorphine from nor-buprenorphine by reductive alkylation which results the product in high purity due to milder reaction conditions and compatibility with phenol function as well.

The best yield of buprenorphine starting from orvinol reaches up to 73%.

An optimized synthesis is described below purely for illustrative purposes: 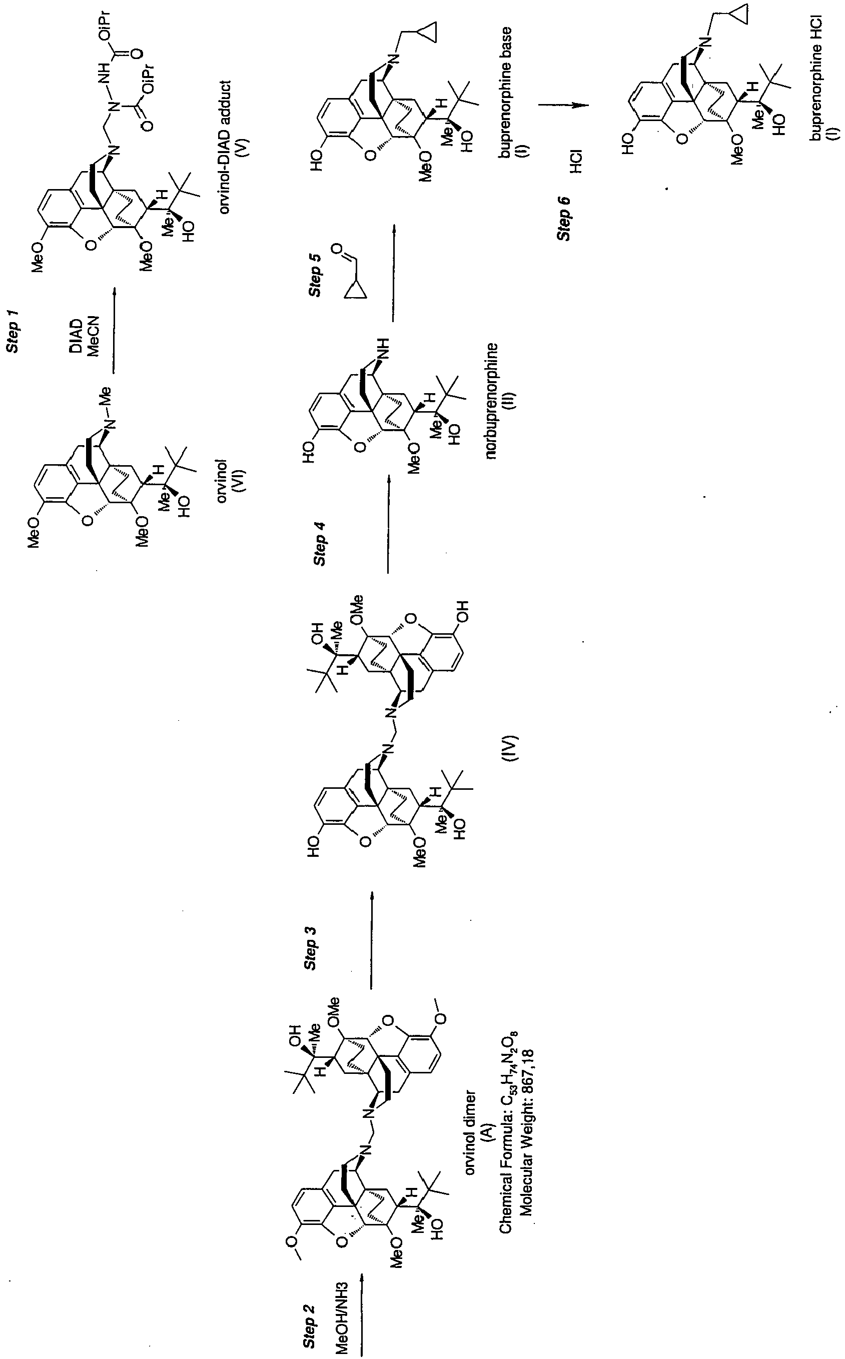
Steps 1-2 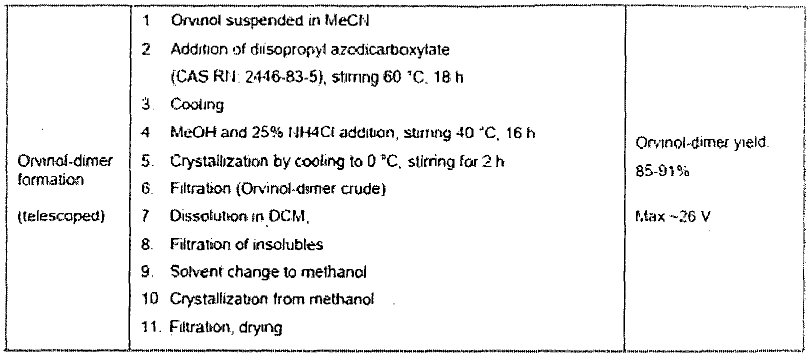
Steps 3-4 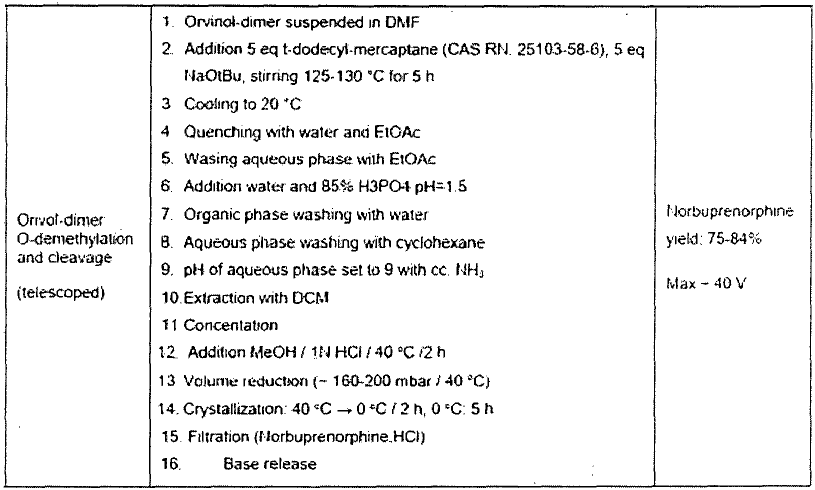
Step 5 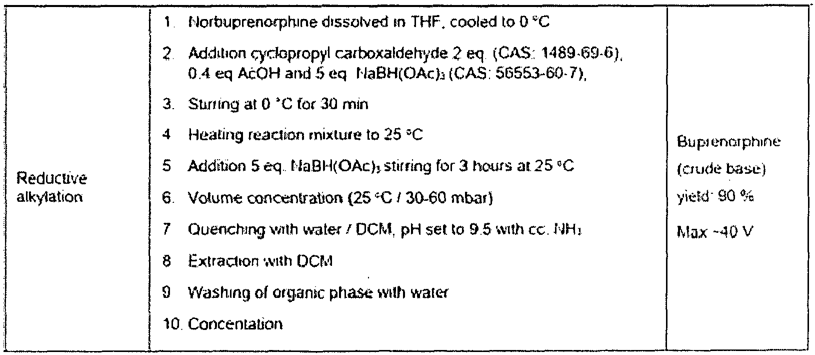
Step 6

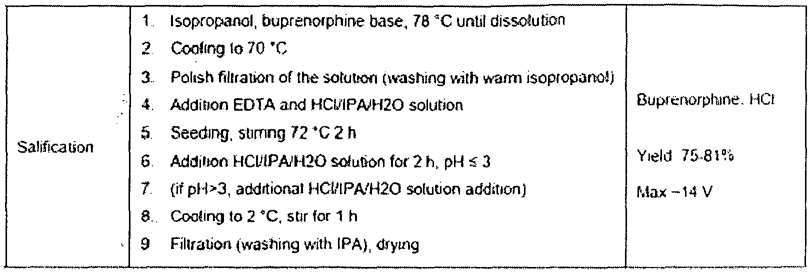

The analytical methods were used to monitor the reactions:
**HPLC impurity method of buprenorphine and buprenorphine hydrochloride**
Instrument: HPLC with UV/VIS or DAD detector with auto sampler or equivalent.
Column: Kinetex 100A, C18, 2.6 µm 150 × 4.6 mm or equivalent.

### Test conditions

Column temperature 30 °C
Flow rate 1.3 mL/min
Injection volume 5.0 µL
Autosampler temperature 25 °C
Detection 240 nm, (288 nm)
Analysis time 35 min
Sample concentration 0.5 mg/mL

### Gradient program:

| Time [min] | Mobile phase A [% V/V] | Mobile phase B [% V/V] |
|---|---|---|
| Initial | 89 | 11 |
| 2.00 | 89 | 11 |
| 12.00 | 64 | 36 |
| 15.00 | 41 | 59 |
| 20.00 | 39 | 61 |
| 25.00 | 89 | 11 |
| 35.00 | 89 | 11 |

### Solutions:

Buffer solution (potassium dihydrogen phosphate):
Weight appr. 5.44 g of potassium dihydrogen phosphate and dissolve in 1000 mL water (pH=4.9).
Mobile phase A: Buffer solution / Acetonitrile = 900 / 100
Mobile phase B: Acetonitrile
Solvent: Methanol
Blank solution: solvent.

### LC-MS method for structure verification of buprenorphine and its impurities

Instrument: UHPLC with UV/VIS or DAD detector with autosampler equipped with MS detector or
equivalent.

Column: Acquity UPLC BEH C8, 1.7 µm 100 × 3.0 mm or equivalent.

### Test conditions

### MS parameters:

| Instrument | Acquity QDa |
|---|---|
| Interface | ES+, ES- |
| Total Ion Current (TIC) Chromatogram | 210 - 400 nm |
| Detected channels | λ₁=240 nm, λ₂=288 nm |
| MS Scan: Mass range | 50 - 600 Da |
| Cone Voltage | 15 V, -15 V |
| Capillary Voltage | Positive: 1.1 kV, Negative: 0.8 kV |

### HPLC parameters:

| Instrument | Acquity H class UPLC system with UVIVIS or DAD detector |
|---|---|
| Column | Acquity UPLC BEH C8, 100 × 3.0 mm × 1.7 µm |
| Column temperature | 30 °C |
| Autosampler temperature | 25 °C |
| Flow rate | 0.5 ml/min |
| Injection volume | 1 µl |
| Mobile Phase A | 2.0 ml of CHOOH / 1000 ml water |
| Mobile Phase B | 2.0 ml of CHOOH / 1000 ml Acetonitrile |
| Diluent | Acetonitrile / Water = 500 / 500 (V/V) + 2.0 mL CHOOH |
| Analysis time | 8 min |
| Sample solution | 0.5 mg / mL |

### Gradient program:

| Time [min] | Mobile phase A [% V/V] | Mobile phase B [% V/V] |
|---|---|---|
| Initial | 95.0 | 5.0 |
| 1.00 | 95.0 | 5.0 |
| 6.00 | 20.0 | 80.0 |
| 6.50 | 20.0 | 80.0 |
| 6.80 | 95.0 | 5.0 |
| 8.00 | 95.0 | 5.0 |

### Solutions

Mobile phase A (0.2% HCOOH in H2O): 2.0 ml of HCOOH / 1000 ml water
Mobile phase B (0.2% HCOOH in ACN): 2.0 ml of HCOOH / 1000 ml Acetonitrile
Solvent (0.2% HCOOH in ACN/H2O): Acetonitrile / Water = 500 / 500 (V/V) + 2.0 mL CHOOH
Blank solution: Solvent.

## Claims

1. Process of preparation of buprenorphine or a derivative thereof of formula (I) : 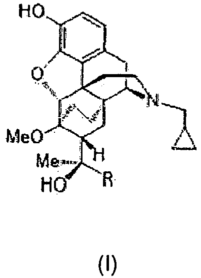 or a pharmaceutically acceptable salt thereof
**characterized in that** said process involves the compound of formula (A) : 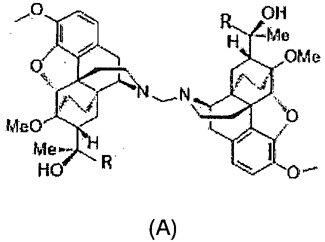
where R is a C1-C6 alkyl.

2. The process according to claim 1 wherein the compound of formula (A) is produced by reacting orvinol or a derivative thereof of formula (VI) : 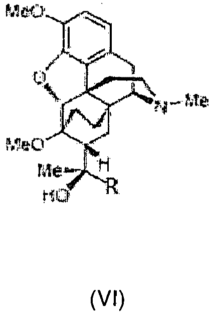 Where R is a C1-C6 alkyl with an azodicarboxylate agent, and reacting the obtained azodicarboxylate adduct with methanol in the presence of aqueous ammonia.

3. The process according to claim 2 wherein the two steps are conducted in a one pot reaction.

4. The process according to claim 2 or 3 wherein the azodicarboxylate agent is diisopropyl azodicarboxylate (DIAD) and the orvinol-azodicarboxylate adduct is of formula (III) : 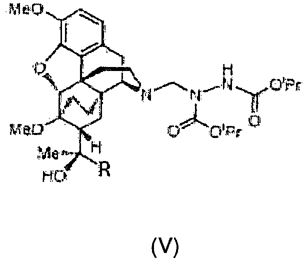 Where
R is a C1-C6 alkyl.

5. The process according to any one of claims 1 to 4 wherein compound A is then crystallized.

6. The process according to claim 5 wherein crystallization is performed in methanol.

7. The process according to anyone of claims 1 to 6 comprising the step of preparing the compound of formula (II) by O-demethylating and cleaving the compound of formula (A) : 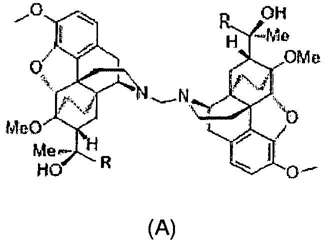 Where
R is a C1-C6 alkyl.

8. The process according to claim 7 wherein the O-demethylation of compound (A) is carried out by reacting the compound of formula (A) with a thiol compound in the presence of a base.

9. The process according to claim 8 wherein the O-demethylation step is conducted in the presence of t-dodecanthiol and NaOtBu.

10. The process according to anyone of claims 7 to 9 wherein the cleaving step is conducted in the presence of an organic solvent in acidic conditions.

11. The process according to claim 10 wherein the organic solvent is MeOH.

12. The process according to claim anyone of claims 7 to 11 wherein the O-demethylation step and the cleaving step are conducted in a one pot reaction.

13. The process according to anyone of claims 7 to 12 wherein the cleaving step is carried out on the isolated O-demethylated dimer of formula (IV) resulting from the O-demethylation step : 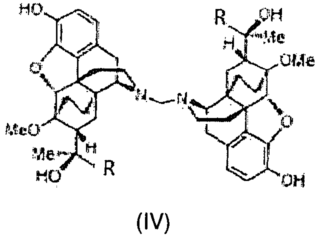 Where
R is a C1-C6 alkyl.

14. The process according to any one of claims 1 to 13 wherein said process comprises
the step of N-alkylating the compound of formula (II): 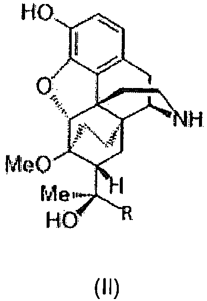
Under reductive conditions with a compound of formula (III) : 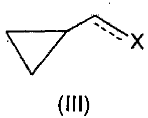
Where
R is a C1-C6 alkyl ;
represents a single or double bond ;
X is chosen from halogen atoms such as Chlorine (CI), Bromine (Br) or Iodine (I) atom, an oxygen atom, an hydroxy group, a tosylate group, a mesylate group, and a sulfonate group;
provided that when X is an oxygen atom, then represents a double bond and when X represents a halogen atom, an hydroxy group, a tosylate group, a mesylate group or a sulfonate group, then represents a single bond, so as to form the compound of formula (I).

15. The process according to any one of claims 1 to 14 further comprising the additional step of forming the pharmaceutically acceptable salt from the compound of formula (I).

16. The process according to claim 14 or 15 wherein the compound of formula (III) is the compound of formula (III') :

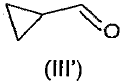

17. A compound of formula (I) 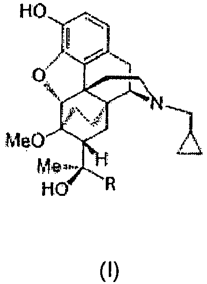 where R is a C1-C6 alkyl,
or a pharmaceutically acceptable salt thereof,
obtainable by the process according to anyone of the preceding claims.

18. A compound of formula (I) according to any of claims 1 to 16 **characterized in that** it has a purity of at least 99% (weight).

19. Pharmaceutical composition comprising a compound of formula (I) according to claims 17 or 18, and a pharmaceutically acceptable excipient.

20. The pharmaceutical composition according to claim 19 suitable for oral, sublingual, or transdermal administration or for injection.

21. A combination comprising buprenorphine according to anyone of claims 17 to 20 and an opioid antagonist, such as naloxone for simultaneous, separate or staggered over time administration.

22. A compound of formula (I) according to anyone of claims 17 to 20 for use for treating and/or preventing opioid use disorder, acute pain and/or chronic pain.

23. A compound of formula (I) for use according to claim 22 which is buprenorphine wherein it is administered at a dose comprised between 2 and 24 mg a day.

24. The compound of formula (A) : 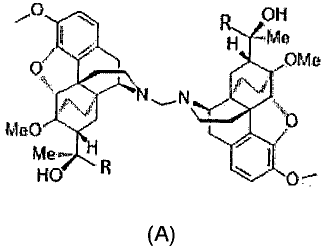 Where
R is a C1-C6 alkyl.

25. The compounds of formula (IV) and (V) : 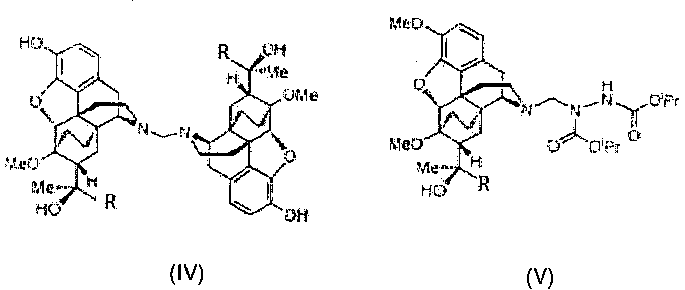 Where
R is a C1-C6 alkyl.

26. A process or compound according to any one of the preceding claims wherein R is *t*-butyl.
